# EUROPEAN PATENT SPECIFICATION

(11) **EP 0 440 787 B1**
(45) Date of publication and mention of the grant of the patent: **13.10.1999**
(21) Application number: 90916668.8
(22) Date of filing: 14.05.1990
(51) Int. Cl.: C07D 237/34, A61K 31/50

(54) **COMPOUNDS OF 1,4-BIS(AMINO)BENZO(G)PHTHALAZENE**
VERBINDUNGEN DER 1,4-BIS(AMINO)BENZO(G)PHTHALAZENREIHE
COMPOSES DE 1,4-BIS(AMINO)BENZO(G)PHTALAZINE

(30) Priority: 19.05.1989 ES 8901705
(43) Date of publication of application: 14.08.1991
(73) Proprietor: CONSEJO SUPERIOR DE INVESTIGACIONES CIENTIFICAS, 28006 Madrid (ES)
(72) Inventor: NAVARRO TORRES, Pilar, Instituto Quimica Medica, Juan de la Cierva, E-28006 Madrid (ES); CAMPAYO PEREZ, Lucrecia, Dep. de Quimica Organica, de Madrid, E-28040 Madrid (ES); ALVAREZ RODRIGUEZ, Ismael, Dep. de Bioquimica Onco, E-28040 Madrid (ES); ESCARIO GARCIA TREVIJANO, José Antonio, Dep. de, de Madrid, E-28040 Madrid (ES)
(74) Representative: Ungria Lopez, Javier
(86) International application number: ES9000016
(87) International publication number: WO9014339

(56) References cited:
- European Journal of Medicinal Chemistry, Chimie Therapeutique, Volumen XXI, No 2, 1986 L. CAMPAYO et al.: "Synthesis and Cytostatic Activity of 1,4-Bis-(Alkylamino)Benzo(g)Phthalazines with Complexing Properties" paginas 143-149
- J. Chem. Soc. Perkin Trans II, 1987, L. CAMPAYO et al.: "Protonation of 1,4-Bis(Alkylamino)Benzo(g)Phthalazine. Crystal Structure of Di-(1,4-Bis-(3-Methoxypropylamino)-3(2)H-Benzo(g)Phthalazinium)Tetrachloro-Cobaltate Monohydrate" pagina s 569-573
- CHEMICAL ABSTRACTS, Volumen 106, 1987, (Columbus, Ohio, US) ver pagina 477* Resumen 5067u & ES, A, 533464 (Consejo Superior de Investigaciones Cientificas) 1 Agosto 1985*

## Description

The present invention provides compounds such as certain 1,4-bis(alkylamino)benzo[g]phthalazines which are useful as anticancer and antiparasitic agents.

### Novel 1,4-bis(amino)benzo[g]phthalazine compounds

### Backgroung of the invention

Daunomycin and Adriamycin play an important role in the clinical treatment of malignant diseases (1). However, these drugs possess cardiotoxicity which was attributed to their aminosugar moiety (2). Many research efforts have been directed towards developing new DNA complexing agents in which the above mentioned side effects would be minimized. Attention has been directed to the anthraquinone and amino moieties of adriamycin as the most likely sites for its known intercalative binding to DNA. Therefore, various flat tricyclic aromatic systems with basic side chains instead of daunosamine have been obtained including ametantrone and mitoxantrone which exhibit intercalating porperties and excellent antineoplasic activity with diminished side effects (3). As the quinone moiety of the above drugs may lead to lipid peroxidation and DNA lesions in cardiac tissue (4), other highly active amentantrone analogues of reduced quinonic character have been reported (5). With the above mentioned aim, we have previously reported a new synthetic procedure of 1,4-bis(alkylamino)benzo[g]phthalazines (6) whose aromatic system containing two of the four conjugated nitrogen atoms as part of the ring with other two in an exocyclic amino group, is easily protonable (7) and shows strong intercalating properties toward DNA (8).

We had found that some derivatives of the above mentioned system such as 1,4-bis[(dimethylamino)propylamino]benzo[g] phthalazine and 1,4-bis[(dimethylamino)propylamino]-6-methoxy-benzo[g]phthalazine were powerful cytostatic compounds (9).

Now we have discovered that other derivatives of 1,4-bis(amino)benzo[g]phthalazine not bearing basic nitogen atoms linked to the terminal carbons of their alkylamino chains are also powerful cytostatic agents and show antitumoral and antiparasitic activities.

The present invention provides novel compounds of the formula I 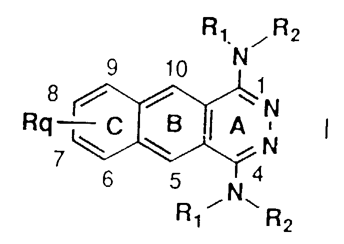 wherein,
the aryl moiety is mono- or di-substituted at any feasible position(s) in the ring C (when q is 1 or 2 respectively) or it is unsubstituted (when q is 0),
R is lower alkyl being a branched or linear alkyl group comprised of 1 to 6 carbon atoms, aryl, alkoxy, nitro, amino, alkylamino dimethylamino or acylamino,
R₁ and R₂ are the same or different and are each hydrogen, a lower alkyl being a branched or linear alkyl group of 1 to 6 carbon atoms, aryl, aralkyl, cycloalkyl or cycloalkylalkyl, or R₁ and R₂ taken together with the nitrogen to which they are attached are pyrrolidino, or piperidino.

As used herein "lower alkyl" refers to a linear or branched alkyl group of 1 to 6 carbon atoms.

The term "aryl" refers to an aromatic group.

The term "aralkyl" refers to an aromatic ring attached to the nitrogen atoms by a linear or branched alkyl bridge.

The term "cycloalkyl" refers to a saturated or partially unsaturated monocyclic hydrocarbon.

Compounds of the formula I can be employed as free aromatic bases or as 1,4-bis(amino)benzo[g]phthalazinium salts, or 2(3)-ethyl-1,4-bis(amino)benzo[g]phthalazinium salts of the general formula II 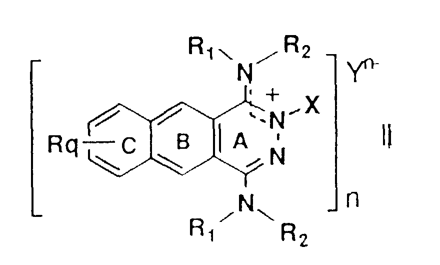 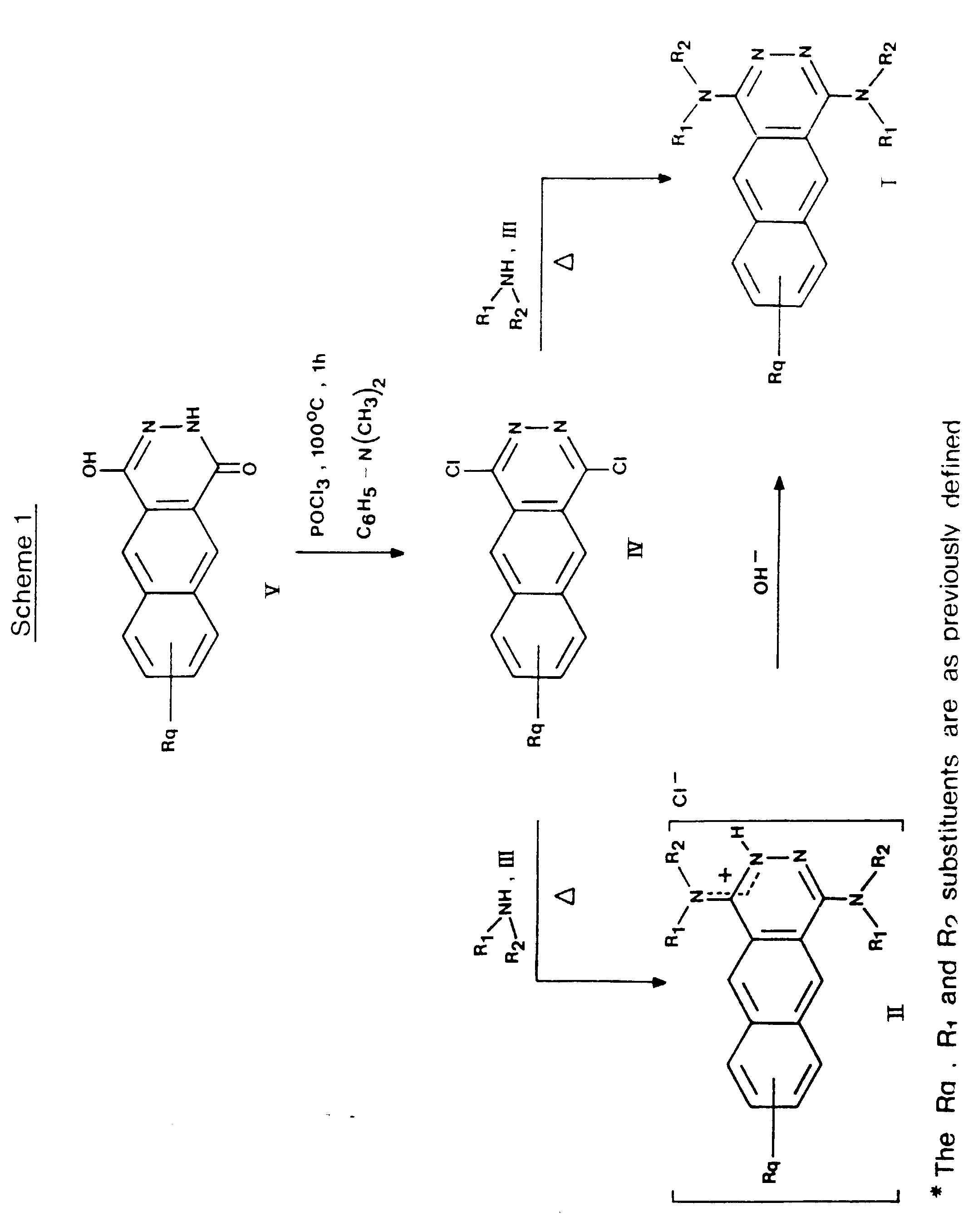 wherein Rq, R₁ and R₂ are as defined above, n is 1 or 2, x is hydrogen atom, methyl or ethyl group and yⁿ⁻ is chloride, bromide, iodide, an oxoacid anion or an organic acid anion.

For example those compounds of formula II prepared from I by treatment of acids such as hydrochloric, sulfuric, oxacid and the like or those compounds prepared by addition of methyl halide or ethyl halide using conventional methods well known in the art.

In general, compounds of formula I may be prepared by a chemical procedure previously patented in Spain by us (6) according to the general synthetic route outlined in Scheme 1.

Compounds of formula I can be prepared by reacting the appropriately substituted benzo[g]phthalhydrazide V with phosphorous oxychloride and dimethylaniline to give the corresponding 1,4-dichloro-benzo[g]phthalazine IV, which is further reacted with the appropiate amine III, in enough amount to act as reactive, solvent and aceptor of hydracid acid to give the corresponding 1,4-bis(amino)-benzo[g]phthalazine compound.

In function of a variety of factors from the reaction mixture the expected free base of formula I or its monohydrochloride of formula II it can be obtained. The latter by treatment in a basic medium affords the corresponding free base of formula I.

The following examples serve to illustrate synthetic procedures to make compounds of the formula I according to the procedure outlined in Scheme 1. These examples are intented to be illustrative only.

### EXAMPLE 1

### 1,4-bis(n-butylamino)benzo[g]phthalazine I (q = 0; R = H; R₁ = H; R₂ = -(CH₂)₃CH₃

A mixture of 1,4-dichlorobenzo[g]phthalazine (9.23 mmol) and n-butylamine (20 mL) was heated in an autoclave at 130°C for 12 h. After cooling to room temperature, the excess of n-butylamine was evaporated to dryness under vacuum. The residue was extracted with chloroform and the resulting solution treated with 5% aqueous solution of sodium hydroxide (100 mL). After the chloroformic layer was separated and the organic solvent removed, the residue was purified by flash chromatography on silica gel Merck (200-400 mesh) using a mixture of n-hexane, ethyl acetate, methanol (v/v 1:1:0.3) as eluent. Removal of solvents from the fraction of Rf = 0.11 afforded a yellow solid which was dissolved in chloroform and filtered through a column of basic aluminium oxide Merck. Removal of the chloroform to dryness afforded 1.1 gr (R = 37%) of 1,4-bis(n-butylamino)benzo[g]phthalazine. m.p. 177-179°C having the following physical properties: IR(KBr) νₘₐₓ, 3300, 3050, 2950, 2910, 2850, 1620, 1495, 1425, 1360, 1220, 1140, 880, 745, 675 cm⁻¹. ¹H NMR (DMSO-d₆) δ, 8.81 (s, 2H, H₅ and H₁₀); 8.10 (m, 2H, H₆ and H₉); 7.68 (m, 2H, H₇ and H₈), 6.44[m, 2H, NH (disappears with D₂O)]; 3.44 (t, 4H, 1'-CH₂-N), 1.69 (m, 4H, 2'-CH₂); 1.42 (m, 4H, 3'-CH₂), 0.94 (t, 6H, 4'-CH₃) ppm.

MS (m/z): 323 (M⁺+1,17), 322 (M⁺, 61), 279 (100).

| Analysis: | | | |
|---|---|---|---|
| Calcd for C₂₀H₂₆N₄ | C, 74.49 | H, 8.12 | N, 17.37 |
| Found | C, 74.78 | H, 7.96 | N, 17.31 |

### EXAMPLE 2

### 1,4-bis(n-butylamino)benzo[g]phthalazine monohydrochloride II [q = 0; R = H; R₁ = H; R₂ = (CH₂)₃CH₃, n = 1, X = H; Y = Cl]

A mixture of 1,4-dichlorobenzo[g]phthalazine (9.23 mmol) and n-butylamine (20 mL) was heated in an autoclave at 130°C for 12 h. After cooling to room temperature, the excess of n-butyl amine was evaporated to dryness under vacuum. When the residue was treated with acetone (100 mL) a yellow solid was formed. After being filtered off and dried it afforded 1.87 g (55% yield) of 1,4-bis(n-butylamino)benzo[g]phthalazine monohydrochloride. m.p. 195-197°C.

IR (KBr) νₘₐₓ, 3320-2600 (3240, 3100, 3000, 2940, 2920, 2850), 1625, 1575, 1545, 1425, 1360, 1325, 1155, 1025, 900, 760, 678 cm⁻¹.

¹H NMR (DMSO-d₆) δ, 9.38 (s, 2H, H₅ and H₁₀), 8.22 (m, 2H, H₆ and H₉), 7.88 (m, 2H, H₇ and H₈), 3.43 (m, 4H, 1'-CH₂-N), 1.93-1.13 (m. broad, 8H, 2'-CH₂ and 3'-CH₂), 0.93 (t, 6H, 4'-CH₃) ppm.

| Analysis | | | | |
|---|---|---|---|---|
| Calcd. for C₂₀H₂₆N₄. 1 HCl. 0.5 H₂O | C, 65.31 | H, 7.62 | N, 15.24 | Cl, 9.64 |
| Found | C, 65,07 | H, 7.73 | N, 15.02 | Cl, 9.54 |

### EXAMPLE 3

### Preparation of 1,4-bis(n-butylamino)-6-methoxy-benzo[g] phthalazine I [q = 1; R = OCH₃ (at C-6); R₁ = H; R₂= ( CH₂)₃CH₃]

A mixture of 1,4-dichloro-6-methoxybenzo[g]phthalazine (3.94 mmol) and n-butylamine (25 mL) was treated in an autoclave at 130°C for 6 h. After cooling to room temperature, the excess of n-butylamine was evaporated to dryness under vacuum and the residue was extracted with chloroform (2 x 50 mL). The resulting extracts were concentrated and purified by flash chromatography on silica gel 60 Merck (200-400 mesh), using a mixture of chloroform, benzene, methanol (v/v, 1: 0.5: 0.2) as eluent. The removal of solvents from the fraction of Rf = 0.11 afforded 0.67 g (48%) of l,4-bis(n-butylamino)-6-methoxy-benzo [g]phthalazine as a brown solid, melting at 178-180°C, which shows the following physical properties:

IR (KBr)νₘₐₓ, 3280, 3050, 2950, 2900, 2850, 1645, 1630, 1560, 1500, 1450, 1360, 1255, 1140, 1020, 790, 740 cm⁻¹.

¹H NMR (DMSO-d₆)δ, 9.06 (s, 1H, H₅); 8.83 (s, 1H, H₁₀), 7.64 (m, 2H, H₈ and H₉), 7.12 (m, 1H, H₇), 6.9-7.5 [m, 2H, NH (disappears with D₂O)], 4.05 (s, 3H, CH₃O), 3.52-3.42 (m, 4H, 1'-CH₂-N), 1.70 (m, 4H, 2'-CH₂), 1.43 (m, 4R, 3'-CH₂), 0.95 (t, 6H, 4'-CH₃) ppm.

MS (m/z): 353 (M⁺+1,23), 352 (M⁺, 67), 309 (100).

| Analysis | | | |
|---|---|---|---|
| Calcd. for C₂₁H₂₈N₄O. 1 MeOH. 0.2 H₂O | C, 68.11 | H, 8.35 | N, 14.45 |
| Found | C, 67.93 | H, 8.43 | N, 14.67 |

By procedures analogous to that described above, the following 1,4-bis-benzo[g]phthalazines can be prepared:
1,4-bis(n-butylamino)-6-dimethylamino-benzo[g]phthalazine
1,4-bis(n-propylamino)benzo[g]phthalazine
1,4-bis(n-propylamino)-6-methoxy-benzo[g]phthalazine
1,4-bis(n-propylamino)-6-dimethylamino-benzo-[g]phthalazine
1,4-bis(ethylamino)benzo[g]phthalazine
1,4-bis(ethylamino)-6-methoxy-benzo[g]phthalazine
1,4-bis(ethylamino)-6-dimethylamino-benzo[g]phthalazine
1,4-bis(methylamino)benzo[g]phthalazine
1,4-bis(methylamino)-6-methoxy-benzo[g]phthalazine
1,4-bis(methylamino)-6-dimethylamino-benzo[g]phthalazine
1,4-bis(amino)benzo[g]phthalazine
1,4-bis(amino)-6-methoxy-benzo[g]phthalazine
1,4-bis(amino)-6-dimethylamino-benzo[g]phthalazine

The starting materials for the above reaction Scheme 1, i.e., the appropriately substituted 2,3-dihydrobenzo[g]phthalazine-1,4-diones (V) and their corresponding 1,4-dichloroderivatives (IV), are readily obtained through the use of commonly available reagents modified if required through standard synthetic schemes, procedures and techniques as are well known and appreciated by those of ordinary skill in the art.

For example the starting 2,3-dihydrobenzo[g]phthalazine-1,4-diones V for compounds of formula I wherein q = 0 or 1 and R is an hydrogen atom or NO₂, OCH₃ or (CH₃)₂N groups substituted at C-6 can be prepared by procedures analogous to that described by P. Navarro et al. (10, 11). The appropriate 1,4-dichlorobenzo[g]phthalazines IV intermediates for compounds of the formula I wherein q = 0 or 1 and R is H atom, NO₂, OCH₃ or N(CH₃)₂ group can be prepared by procedures analogous to that described by P. Navarro et al. (7).

In another embodiment, the compounds of formula I of this invention exhibit antitumoral activity "in vitro" and "in vivo" as well as an excellent tripanosomicide and trichomonacide activity "in vitro".

It is generally believed that there is a correlation between compounds which exhibit antiparasitic activity "in vitro" and the medical effect of being useful in treating parasitic infection in a patient suffering therefrom.

The following compound:
1,4-bis(n-butylamino)benzo[g]phthalazine (BBph) is particularly preferred embodiment of the present invention as is shown by the following Experiments:

### Experiment 1: ANTITUMORAL ACTIVITY

### I. "In vitro" effects":

The cytostatic and cytocidal activity of BBPh has been evaluated employing HeLa cell lines (from a human uterine cervix carcinoma), ADLD-clon (from a human malignant melanona) and a semicontinuos LMMB cell line (from normal human tonsilar fibroblasts).

In order to perform both "in vitro" and "in vivo" experiments a concentrated stock solution of BBPh in dimethyl sulfoxide (DMSO) was prepared. From it increasing diluted solutions in McCoy's R5aI grown medium supplemented with 5% human serum, 5% calf serum, 5% fetal bovine serum, 100 IU/mL penicillin, 100 ug/mL streptomycin and 16 ug/mL gentamycin were obtained.

Serial dilutions of BBPh and the solvent were assayed in cultures seeded 24 hours before on 96-well "Microtiter" plates. The seeding was carried out by depositing 200 µL of cell suspension in growth medium in each well, adjusted to 3 x 10⁵/mL HeLa cells, 9 x 10⁵/mL ADLD-clon cells or 4 x 10⁵/mL LMMB cells.

The plates with different concentrations of BBPh and control solutions were incubated at 37°C in a humidily saturated air containing 5% CO₂.

Then the plates were fixed and stained to stimate the effects of BBPh under the conditions described by Alvarez (1986) (11) taken Adriamycin as antitumoral compound of reference. Under the above mentioned conditions, the 50 per 100 inhibitory dose (ID₅₀) or concentration producing the death or growth inhibition of 50% of the cells of Adriamycin and BBPh were very close as it is indicated below:

| Cell Cultures | ID₅₀(µg/mL) | |
|---|---|---|
| | BBPh | Adriamycin |
| HeLa | 0.67 | 0.33 |
| ADLD-clon | 0.67 | 0.33 |
| LMMB | 1.08 | 0.07 |

It was also observed that the morphology adopted by the cells exposed to inhibitory doses is clearly different for cells treated with Adriamycin or BBPh. Adriamycin inhibited cells showing very big nuclei, indicating that growth inhibition is produced after the S phase has taken place. However, cells inhibited by BBPh show always small nuclei possible because the grow stop is produced before the S phase.

It is interesting to note that BBPh is slightly less toxic "in vitro" for normal LMMB cells than for HeLa and ADLD-clon neoplastic cells contrary to what happens in the case of Adriamycin, which show a toxicity slightly higher upon normal than upon the neoplastic cells used in these assays.

### II. "In vivo" effects

Five groups of six 2-month male swiss mice weighing 25 grams were disposed. Each animal received a transplant of 10⁵ Ehrlich ascites cancer cells by the subcutaneous route in the interscapular region. Fourteen days later, when the tumors were evident and measured more than 3 millimeter in diameter, the animals were submitted to the different treatments.

Group 1 received a first intraperitoneal dose of 300 µL of DMSO followed by equal subcutaneous doses, the next three consecutive days.

Group 2 received a single intraperitoneal dose of 100 µg of BBPh solubilized in 300 µL of DMSO, followed by equal subcutaneous doses the next three consecutive days.

Group 3 received a single intraperitoneal dose of 500 µg of BBPh in 300 µL of DMSO.

Group 4 received a single intraperitoneal dose of 1.000 µg of BBPh in 300 µL of DMSO.

Group 5 received a single subcutaneous dose of 4.000 µg BBPh in an oily solution.

Tables 1 and 2 summarize the main data related to the evolution of the tumors and surviving animals of each group.

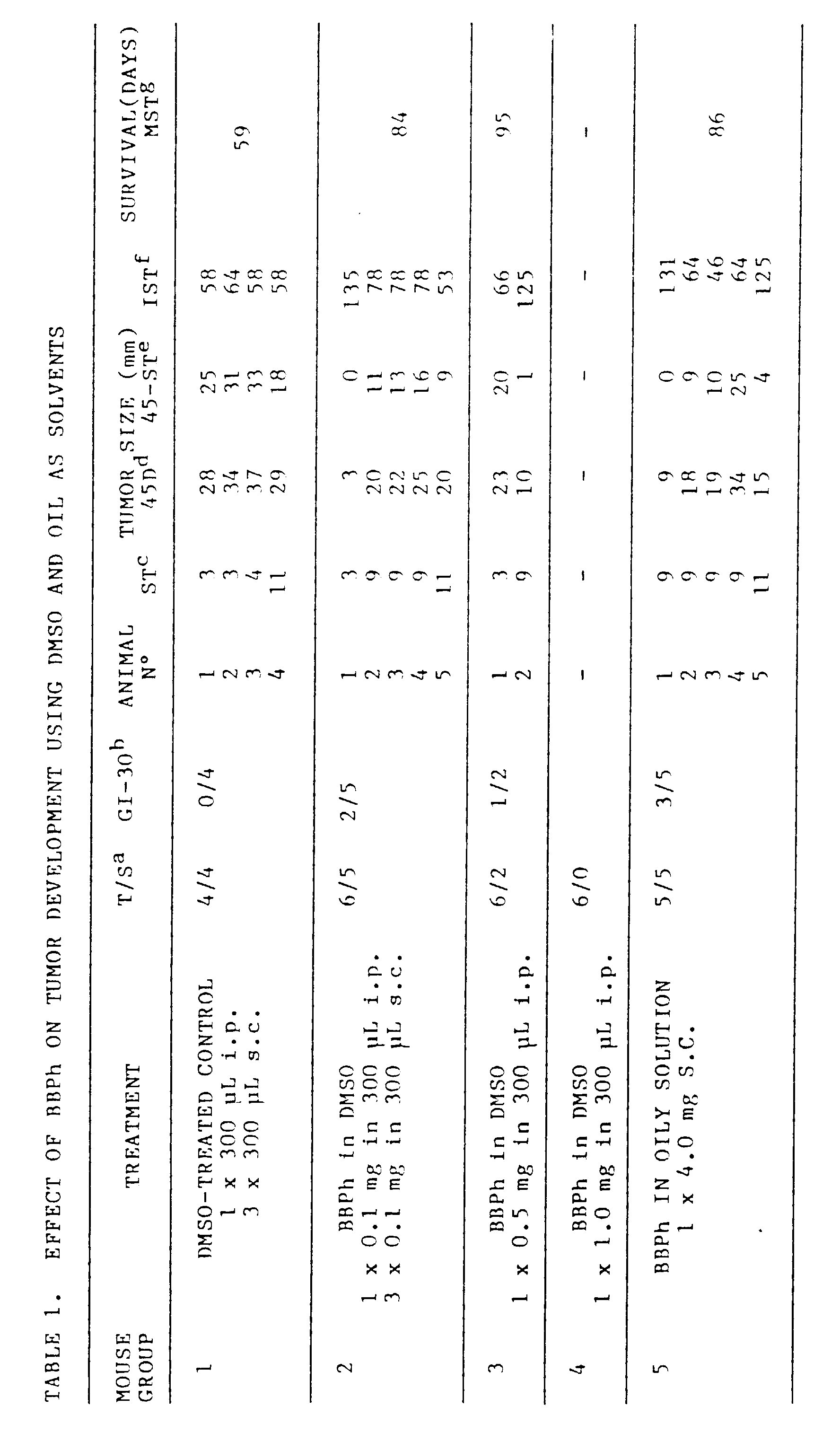

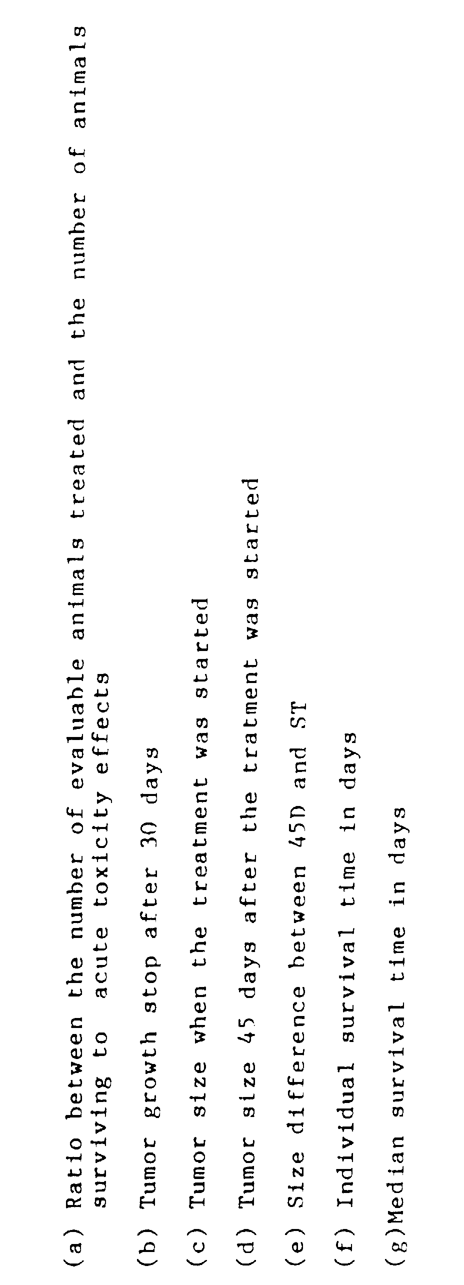

**Table 2.**

| ANTITUMORAL ACTIVITY OF BBPh ON EHRLICH ASCITES CANCER CELLS USING DMSO AS SOLVENT | | | | |
|---|---|---|---|---|
| Drug | Overal Dose (mg/Kg) | TST (days) | T/C^{e} (%) | Toxic death |
| Control^{a} (DMSO-treated) | --- | 59 | --- | 0/6 |
| BBPh | 16^{b} | 84 | 142^{f} | 1/6 |
| BBPh | 20^{c} | 96 | 161^{f} | 4/6 |
| BBPh | 40^{d} | 1 | Toxic | 6/6 |

| | | | | |
|---|---|---|---|---|
| ^{a} A first i.p. dose of 0.3 mL of DMSO followed by similar s.c. doses the next three consecutive days | | | | |
| ^{b} A first i.p. dose of 0.1 mg BBPh solubilized in 0.3 mL of DMSO, followed by similar s.c. doses the next three consecutive days | | | | |
| ^{c} A single i.p. dose of 0.5 mg BBPh in 0.3 mL of DMSO | | | | |
| ^{d} A single i.p. dose of 1.0 mg BBPh in 0.3 mL of DMSO | | | | |
| ^{e} T/C (%) = Median survival of treated animals (T)/ Median survival time of control animals (C) x 100. (A compound may be considered active when T/C% value is > 125%) | | | | |

The data gathered in Table 1 are indicating that BBPh produce a clear inhibition of tumoral growth either using DMSO as solvent (groups 2 and 3) or oily solutions (group 5). Using DMSO as solvent via intraperitoneal (i.p.) in a single dose of 20 mg/Kg the BBPh showed to be toxic. However its 50 per 100 lethal dose (LD₅₀ = 12 mg/Kg) is much lower than those exhibited by many clinical drugs actually used in the treatment of malignant diseases such as Actinomicin D (LD₅₀ = 0.095 mg/Kg), Vincristine (LD₅₀ = 4.5 mg/Kg), Dichloroplatinum II (LD₅₀ = 8.9 mg/Kg) or Daunomycin (LD₅₀ = 9.2 mg/Kg) (12).

Besides when a DMSO solution of BBPh (0.1 mg in 0.3 mL of DMSO) is administered in four consecutive days using a mixed treatment (one dose i.p. followed by three doses s.c.) the toxic deats are drastically reduced (T/S = 6/5) and the median survival time of the treated animals (group 2: MST = 84 days) is considerable higher than the exhibited by the DMSO-treated control (group 1: MST = 59 days).

Furthermore it is interesting to observe that any animal belonging to group 5 which was treated with an oily solution of BBPh administered in a single and high s.c. dose of 160 mg/kg it does not suffered toxic death (T/S = 5/5) and the median survival time was 86 days (table 1).

Considering the above mentioned results together with the T/C% values indicated in table 2 we can assert that BBPh shows a clear antineoplasic activity in vivo against Ehrlich ascites cancer cells.

### Experiment 2. TRYPANOSOMICIDE ACTIVITY

Human Chagas' disease caused by Trypanosoma cruzi affects nearly 12 millions of people in the world and shows a considerable mortality rate associated with new infections. In spite of this until now there is not any effective treatment of the above mentioned disease and only Nifurtimox and Benzonidazole are used with moderate succes (13).

Although Trypanosoma cruzi is usually transmitted by blood sucking vectors, blood transfusion is also an important source of infection in urban areas of endemic zones (14). Consequently, there is an urgent need for the development of new effective medicaments for the direct Chagas' disease treatment as well as for the prevention of its transmision by blood transfusions (15).

Some anticancer drugs such as mitomycin C and antinomycin D which interact with nucleic acids have shown activity against Trypanosoma cruzi (16). Furthermore the trypanosomicide activity exhibited in general by intercalating agents such as phenanthridinium salts (i.e. ethidium bromide) has been related to the quaternization of the phenanthridine nucleous (17). Following a similar behaviour the 1,4-bis(buthylamino)benzo[g] phthalazine (BBPh) which is able to form 1,4-bis(buthylamino) benzo[g]phthalazinium cations at physiological PH (PKa of BBPh = 8.6) and is a potential intercalating agent has shown both, antineoplasic and trypanosomicide activities.

### "In vitro" effects:

The Y. strain of T. cruzi used in this experiment was isolated in 1953 from an infected human and it is defined as "depended cell" strain due to its affinity for invading reticule endotelial cells. It is characterized by the abundance of slender blood forms and by its high virulence in white mice.

In order to evaluate the activity of BBPh, NMRI mice were inoculated with T. Cruzi and exanguinated after 7-10 days by cardiac punture using heparin as anticoagulant. The infected blood was mixed with normal blood and 0.5 mL of the resulting mixture containing a parasite density of 500.000/mL calculated by using the Brener' Method (18) , was mixed with 0.5 mL of a solution of BBPh in phosphate buffer saline (PBS) containing 16% ethylene glycol. Five different doses of 1000, 500, 250, 125 and 75 ug/mL of BBPh were assayed using three identical tubes for each of them taking as reference aditional tubes containing Ct control (0.5 mL of infected blood and 0.5 mL of PBS) and Cd control (0.5 mL of infected blood and 0.5 mL of PBS solution containing 16% of diethylene glycole).

After the above samples were kept in the refrigerator at 4°C and shaked for an incubation period of 24 h, the parasites were again counted.

Under the above mentioned conditions all the samples treated with BBPh showed negative parasitemia (Table 3).

**Table 3.**

| | | | | | | | |
|---|---|---|---|---|---|---|---|
| Trypanosomicide activity of BBPh "in vitro". Examination of NMRI mice blood infected with T. cruzi (500.000/mL) after be treated with BBPh and incubated 24 h at 4°C. | | | | | | | |

| | Cd^{a} | Ct^{b} | BBPh (µg /mL) | | | | |
|---|---|---|---|---|---|---|---|
| | | | 1000 | 500 | 250 | 125 | 75 |
| N° Tryp/mL | 298 | 247 | 0 | 0 | 0 | 0 | 0 |

| | | | | | | | |
|---|---|---|---|---|---|---|---|
| a) 0.5 mL of infected blood and 0.5 mL of a phosphate buffer saline (BBS) solution containing 16% of diethylene glycole | | | | | | | |
| b) 0.5 mL of infected blood and 0.5 mL of PBS) c) 0.5 mL of infected blood and 0.5 mL of a solution of BBPh in PBS containing 16% of ethylene glycole | | | | | | | |

### Experiment 3: TRICHOMONACIDE ACTIVITY

### "In vitro" effects:

The cultures of the strains of Trichomonas vaginalis (Srain G) used as experimental model were grown in Diamond Medium (TYM) without agar (100.000 organisms/mL). The 1,4-bis(buthylamino)benzo[g]phthalazine (BBPh) was added to the cultures at the different preestablished doses 6 h after reseeding (hour 0); counting was made after 24 h and 48 h of contact between the compound and protozoa at 37°C. Minimal inhibition concentration (MIC) and Minimal cytocidal concentration (MCC) were established in accordance with the definitions proposed by Escario et al. (19). The resulting data are the following:

| Compound | Trichomonacidal activity (µg/mL) | |
|---|---|---|
| | MIC | MCC |
| Metronidazole | 4-8 | 8 |
| BBPh | 5-10 | 10-25 |

From the above values it can be observed that the trichomonacide activity of BBPh is very close to the exhibited by metronidazole which was taken as reference in this experiment.

### References and notes

(1) F. Arcamone "Doxorubicin Anticancer Antibiotics", Academic Press, New York (1981)
(2) R. H. Adamson, Cancer Chemother. Rep., 1974, 58, 293.
(3) I. E. Smith, Cancer Treat Rev., 1983, 10, 103.
(4) J. W. Jown, S. M. Sondhi, S. B. Mandal and J. Murphy, J. Org. Chem., 1982, 47, 4304.
(5) H.D.H. Showalter, J. L. Johnson, L. M. Werbel, W. R. Leopold, R. C. Jackson, E. F. Elslager, J. Med. Chem., 1984, 27, 255.
(6) L. Campayo and P. Navarro. Spanish Patent n° 533464 (1984)
(7) L. Campayo, F. H. Cano, C. Foces-Foces and P. Navarro, J. Chem. Soc., Perkin Trans II, 1987, 569-573
(8) E. Giralt, M. Pons, L. Campayo and P. Navarro, Unpublished results.
(9) L. Campayo and P. Navarro, Eur. J. Med. Chem. Chem. Ther. 1986, 21, 143-149.
(10) L. Campayo, B. Jiménez, T. Manzano, P. Navarro, Synthesis, 1985, 197-200.
(11) I. Alvarez, "Trends in Cancer Research S-3", Ed. Servicio Editorial de la Universidad del País Vasco, 1986, p 199-218
(12) F. M. Shabel, D. P. Griswold, T. H. Corbett, W. R. Laster, J. G. Mayo and H. H. Lloyd, "Methods in Cancer Research", vol 17, Cancer Drug Developpment Part B. Ed. by V. T. Devita and H. Bush, New York, Academic Press IN. 1979, p 3-51.
(13) L.S. Filardi, Z. Brener, Trans. R. Soc. Trop. Med. Hyg., 81 (5), 755-9 (1987).
(14) H. M. Souza, C. A. Morais, J. R. Mineo. Rev. da Soc. Brasil de Med. Trop., 1985, 18 (1), 11-15.
(15) D. J. Hammond, J. Hogg, W. E. Gutteridge, Exp. Parasitol., 1985, 60, 32-42.
(16) J. F. Fernandez, M. Halsman and O. Castellani, Nature (London), 1965, 207, 1004-1005.
(17) W. J. Ross in "Chemotherapy of Trypanosomiasis Protozoan Diseases" in "Burger's Medicinal Chemistry" Part II, Ed. by M. E. Wolf, John Willey and Sons Inc. New York.p. 439 (1979).
(18) Z. Brener. Adv. in Pharmacol. and Chemother., 1975, 13, 1-44.
(19) J. A. Escario, A. Sanchez-Souza, C. Gómez-Criado, M. L. Jiménez, E. A. Fernández-Jorg and F. Baquero. Ann. Inst. Pasteur, 1985, 136A, 371.

## Claims (Claims for the following Contracting State(s): AT, BE, CH, DE, DK, FR, GB, IT, LI, LU, NL, SE)

1. A compound of the formula 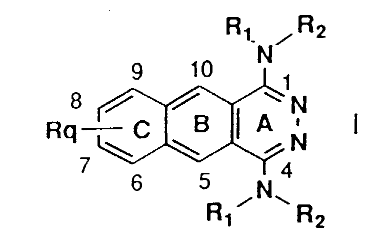 wherein,
the aryl moiety is mono- or di-substituted at any feasible position(s) in the ring C (when q is 1 or 2 respectively) or it is unsubstituted (when q is 0),
R is lower alkyl being a branched or linear alkyl group of 1 to 6 carbon atoms, aryl, alkoxy, nitro, amino, alkylamino, dimethylamino or acylamino,
R₁ and R₂ are the same or different and are each hydrogen, a lower alkyl being a branched or linear alkyl group of 1 to 6 carbon atoms, aryl, aralkyl, cycloalkyl, cycloalkylalkyl, or R₁ and R₂ taken together with the nitrogen to which they are attached are pyrrolidino or piperidino or a 1,4-bis(amino)benzo[g]phthalazinium salt of the formula 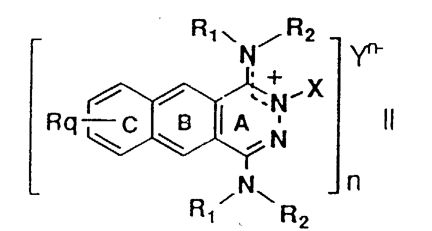 wherein R^{q}, R₁ and R₂ are defined as above, n is 1 or 2, X is hydrogen, a methyl or ethyl group and Yⁿ⁻ is chloride, bromide, iodide, an oxoacid anion or an anorganic anion.

2. A compound according to claim 1 of the formula 1 wherein the aryl moiety is unsubstituted (q is 0), R₁ is hydrogen and R₂ is hydrogen, a lower alkyl being a branched or linear alkyl group of 1 to 6 carbon atoms, aryl, aralkyl, cycloalkyl, cycloalkylalkyl.

3. A compound according to claim 1 of the formula I wherein the aryl moiety is monosubsituted (q is 1), at the C-6 carbon and R is a lower alkyl being a branched or linear alkyl group of 1 to 6 carbon atoms, aryl, alkoxy, nitro, amino, alkylamino or an acylamino group.

4. A compound according to claim 1 or 3 of the formula I wherein R₁ is hydrogen and R₂ is hydrogen, a lower alkyl being a branched or linear alkyl group comprised of 1 to 6 carbon atoms, aryl, aralkyl, cycloalkyl or cycloalkylalkyl.

5. A compound according to claim 1 of the formula II wherein the aryl moiety is unsubstituted (q is 0), X is hydrogen, a methyl or ethyl group, n is 1 or 2 and Yⁿ⁻ is chloride, bromide, iodoide, an oxoacid anion or an organic acid anion.

6. A compound according to any of claims 1-5 wherein the aryl moiety is monosubstituted at the C-6 position in ring C (when q is 1), or unsubstituted (when q is 0), R is methoxy or dimethylamino, R₁ and R₂ are hydrogen, an alkyl group comprised of 1 to 6 carbon atoms in linear, branched or cyclic configuration.

7. 1,4-bis(n-buthylamino)benzo[g]phthalazine.

8. 1,4-bis(n-buthylamino)-3(2)H-benzo[g]phtalazinium chloride.

9. 1,4-bis(n-buthylamino)-6-methoxy-benzo[g]phthalazine.

10. 1,4-bis(n-buthylamino)-6-methoxy-benzo[g]phthalazinium chloride.

11. 1,4-bis(n-propylamino)-benzo[g]phthalazine.

12. 1,4-bis(n-propylamino)-3(2)H-benzo [g]phthalazinium chloride.

13. 1,4-bis(n-propylamino)-6-methoxy-benzo[g]phthalazine.

14. 1,4-bis(n-propylamino)-6-methoxy-3(2)H-benzo[g]phthalazinium chloride.

15. A pharmaceutical composition comprising a compound of any of the preceding claims in a mixture with a pharmaceutically acceptable carrier.

16. A process for preparing a compound of the formula 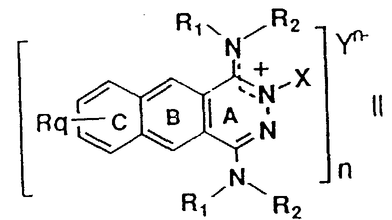 wherein
the aryl moiety is mono- or di-substituted at any feasible position(s) in the ring C (when q is 1 or 2 respectively) or it is unsubstituted (when q is 0),
R is lower alkyl being a branched or linear alkyl group of 1 to 6 carbon atoms, aryl, alkoxy, nitro, amino, alkylamino, dimethylamino or acylamino,
R₁ and R₂ are the same or different and are each hydrogen, a lower alkyl being a branched or linear alkyl group of 1 to 6 carbon atoms, aryl, aralkyl, cycloalkyl, cycloalkylalkyl, or R₁ and R₂ taken together with the nitrogen to which they are attached are pyrrolidino, or piperidino, n is 1 or 2, X is hydrogen, a methyl or ethyl group, and Yⁿ⁻ is chloride, bromide, iodide, an oxoacid anion or an anorganic anion, which comprises reacting a 1,4-bis(amino)benzo[g]phthalazine of the formula 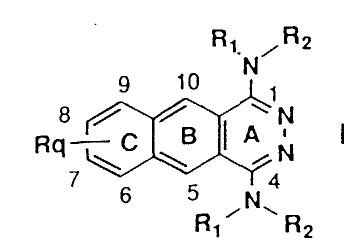 wherein R_{q}, R₁ and R₂ are defined as above, with acids such as hydrochloric, sulfuric acid and the like or alkyl halides such as methyl or ethyl chloride.

17. A process according to claim 16 for preparing a compound of formula II wherein the aryl moiety is unsubstituted (q is 0), X is hydrogen atom, a methyl or ethyl group, n is 1 or 2, and Yⁿ⁻ is chloride, bromide, iodide, an oxoacid anion or an organic acid anion.

18. A process according to claim 16 for preparing a compound of claims 16 or 17 wherein the aryl moiety is monosubstituted at the C-6 position in the ring C (when q is 1) or unsubstituted (when q is 0), R is hydrogen atom, a methoxy or dimethylamino group, R₁ is hydrogen, R₂ is hydrogen atom, or an alkyl group of 1 to 6 carbons in linear, branched or cyclic configuration.

19. A process according to claim 16 for preparing 1,4-bis(n-buthylamino)-3(2)H-benzo[g]phthalazinium chloride.

20. A process according to claim 16 for preparing 1,4-bis(n-buthylamino)-6-methoxy-3(2)H-benzo[g]phthalazinium chloride.

21. A process according to claim 16 for preparing 1,4-bis(n-propylamino)-3(2)H-benzo[g]phthalazinium chloride.

22. A process according to claim 16 for preparing 1,4-bis(n-propylamino)-3(2)H-6-methoxy-benzo[g]phthalazinium chloride.

23. Use of a compound of any one of claims 1-15 for preparing a medicament for treating cancer.

24. Use of a compound of any one of claims 1-15 for preparing a medicament for treating the Chagas' disease.

25. Use of a compound of any one of claims 1-15 for preparing a medicament for treating trypanosomiasis and other Protozoan diseases.

## Claims (Claims for the following Contracting State(s): ES)

1. A process for preparing a compound of the formula 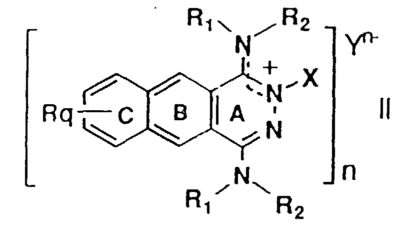 wherein
the aryl moiety is mono- or di-substituted at any feasible position(s) in the ring C (when q is 1 or 2 respectively) or it is unsubstituted (when q is 0),
R is lower alkyl being a branched or linear alkyl group of 1 to 6 carbon atoms, aryl, alkoxy, nitro, amino, alkylamino, dimethylamino or acylamino,
R₁ and R₂ are the same or different and are each hydrogen, a lower alkyl being a branched or linear alkyl group of 1 to 6 carbon atoms, aryl, aralkyl, cycloalkyl, cycloalkylalkyl, or R₁ and R₂ taken together with the nitrogen to which they are attached are pyrrolidino, or piperidino, n is 1 or 2, X is hydrogen, amethyl or ethyl group and Yⁿ⁻ is chloride, bromide, iodide, an oxoacid anion or an anorganic anion, which comprises reacting a 1,4-bis(amino)benzo[g]phthalazine of the formula 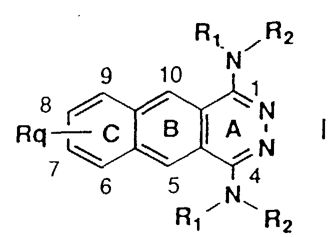 wherein R_{q}, R₁ and R₂ are defined as above, with acids such as hydrochloric, sulfuric acid and the like or alkyl halides such as methyl or ethyl chloride.

2. A process according to claim 1 for preparing a compound of formula II wherein the aryl moiety is unsubstituted (q is 0), X is hydrogen atom, a methyl or ethyl group, n is 1 or 2, and Yⁿ⁻ is chloride, bromide, iodide, an oxoacid anion or an organic acid anion.

3. A process according to claim 1 for preparing a compound of claims 1 or 2 wherein the aryl moiety is monosubstituted at the C-6 position in the ring C (when q is 1) or unsubstituted (when q is 0), R is hydrogen atom, a methoxy or dimethylamino group, R₁ is hydrogen, R₂ is hydrogen atom, or an alkyl group of 1 to 6 carbons in linear, branched or cyclic configuration.

4. A process according to claim 1 for preparing 1,4-bis(n-buthylamino)-3(2)H-benzo[g]phthalazinium chloride.

5. A process according to claim 1 for preparing 1,4-bis(n-buthylamino)-6-methoxy-3(2)H-benzo[g]phthalazinium chloride.

6. A process according to claim 1 for preparing 1,4-bis(n-propylamino)-3(2)H-benzo[g]phthalazinium chloride.

7. A process according to claim 1 for preparing 1,4-bis(n-propylamino)-3(2)H-6-methoxy-benzo[g]phthalazinium chloride.

## Patentansprüche (Patentansprüche für folgende(n) Vertragsstaat(en): AT, BE, CH, DE, DK, FR, GB, IT, LI, LU, NL, SE)

1. Verbindung mit der Formel 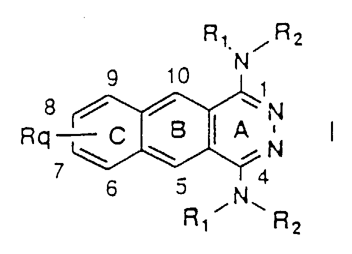 worin
der Arylbestandteil in (einer) beliebigen geeigneten Stellung bzw. Stellungen in dem Ring C mono- oder disubstituiert ist (wenn q 1 bzw. 2 ist) oder unsubstituiert ist (wenn q 0 ist),
R ein niederes Alkyl, bei dem es sich um eine verzweigte oder lineare Alkylgruppe mit 1 bis 6 Kohlenstoffatomen handelt, Aryl, Alkoxy, Nitro, Amino, Alkylamino, Dimethylamino oder Acylamino ist,
R₁ und R₂ gleich oder voneinander verschieden sind und jeweils Wasserstoff, ein niederes Alkyl, bei dem es sich um eine verzweigte oder lineare Alkylgruppe mit 1 bis 6 Kohlenstoffatomen handelt, Aryl, Aralkyl, Cycloalkyl, Cycloalkylalkyl sind, oder R₁ und R₂ zusammen mit dem Stickstoff, an den sie gebunden sind, Pyrrolidino oder Piperidino sind,
oder ein 1,4-Bis(amino)benzo[g]phthalaziniumsalz der Formel 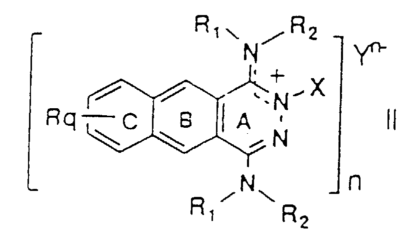 worin R^{q}, R₁ und R₂ wie oben definiert sind, n 1 oder 2 ist, X Wasserstoff, eine Methyl- oder Ethylgruppe ist und Yⁿ⁻ Chlorid, Bromid, lodid, ein Sauerstoffsäureanion oder ein Anion einer organischen Säure ist.

2. Verbindung nach Anspruch 1 mit der Formel I, worin der Arylbestandteil unsubstituiert ist (q ist 0), R₁ Wasserstoff ist und R₂ Wasserstoff, ein niederes Alkyl, bei dem es sich um eine verzweigte oder lineare Alkylgruppe mit 1 bis 6 Kohlenstoffatomen handelt, Aryl, Aralkyl, Cycloalkyl, Cycloalkylalkyl ist.

3. Verbindung nach Anspruch 1 mit der Formel I, worin der Arylbestandteil am C-6-Kohlenstoff monosubstituiert ist (q ist 1), und R ein niederes Alkyl, bei dem es sich um eine verzweigte oder lineare Alkylgruppe mit 1 bis 6 Kohlenstoffatomen handelt, Aryl, Alkoxy, Nitro, Amino, Alkylamino oder eine Acylaminogruppe ist.

4. Verbindung nach Anspruch 1 oder 3 mit der Formel I, worin R₁ Wasserstoff ist und R₂ Wasserstoff, ein niederes Alkyl, bei dem es sich um eine verzweigte oder lineare Alkylgruppe mit 1 bis 6 Kohlenstoffatomen handelt, Aryl, Aralkyl, Cycloalkyl oder Cycloalkylalkyl ist.

5. Verbindung nach Anspruch 1 mit der Formel II, worin der Arylbestandteil unsubstituiert ist (q ist 0), X Wasserstoff, eine Methyl- oder Ethylgruppe ist, n 1 oder 2 ist und Yⁿ⁻ Chlorid, Bromid, lodid, ein Sauerstoffsäureanion oder ein Anion einer organischen Säure ist.

6. Verbindung nach einem der Ansprüche 1 bis 5, worin der Arylbestandteil in der C-6-Stellung im Ring C monosubstituiert ist (wenn q 1 ist) oder unsubstituiert ist (wenn q 0 ist), R Methoxy oder Dimethylamino ist, R₁ und R₂ Wasserstoff, eine Alkylgruppe mit 1 bis 6 Kohlenstoffatomen in linearer, verzweigter oder cyclischer Konfiguration sind.

7. 1,4-Bis(n-butylamino)-benzo[g]phthalazin.

8. 1,4-Bis(n-butylamino)-3(2)H-benzo[g]phthalaziniumchlorid.

9. 1,4-Bis(n-butylamino)-6-methoxy-benzo[g]phthalazin.

10. 1,4-Bis(n-butylamino)-6-methoxy-benzo[g]phthalaziniumchlorid.

11. 1,4-Bis(n-propylamino)-benzo[g]phthalazin.

12. 1,4-Bis(n-propylamino)-3(2)H-benzo[g]phthalaziniumchlorid.

13. 1,4-Bis(n-propylamino)-6-methoxy-benzo[g]phthalazin.

14. 1,4-Bis(n-propylamino)-6-methoxy-3(2)H-benzo[g]phthalaziniumchlorid.

15. Pharmazeutische Zusammensetzung umfassend eine Verbindung nach einem der vorangehenden Ansprüche in einem Gemisch mit einem pharmazeutisch annehmbaren Träger.

16. Verfahren zum Herstellen einer Verbindung mit der Formel 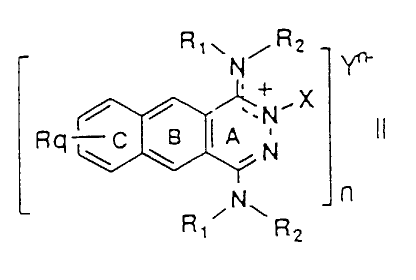 worin
der Arylbestandteil in (einer) beliebigen geeigneten Stellung bzw. Stellungen in dem Ring C mono- oder disubstituiert ist (wenn q 1 bzw. 2 ist) oder unsubstituiert ist (wenn q 0 ist),
R ein niederes Alkyl, bei dem es sich um eine verzweigte oder lineare Alkylgruppe mit 1 bis 6 Kohlenstoffatomen handelt, Aryl, Alkoxy, Nitro, Amino, Alkylamino, Dimethylamino oder Acylamino ist,
R₁ und R₂ gleich oder voneinander verschieden sind und jeweils Wasserstoff, ein niederes Alkyl, bei dem es sich um eine verzweigte oder lineare Alkylgruppe mit 1 bis 6 Kohlenstoffatomen handelt, Aryl, Aralkyl, Cycloalkyl, Cycloalkylalkyl sind, oder R₁ und R₂ zusammen mit dem Stickstoff, an den sie gebunden sind, Pyrrolidino oder Piperidino sind, n 1 oder 2 ist, X Wasserstoff, eine Methyl- oder Ethylgruppe ist, und Yⁿ⁻ Chlorid, Bromid, lodid, ein Sauerstoffsäureanion oder ein Anion einer organischen Säure ist,
welches das Umsetzen eines 1,4-Bis(amino)benzo[g]phthalazins mit der Formel 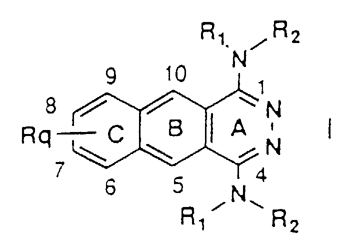 worin R_{q}, R₁ und R₂ wie oben definiert sind, mit Säuren wie Chlorwasserstoffsäure, Schwefelsäure und dergleichen oder Alkylhalogeniden wie Methyl- oder Ethylchlorid umfaßt.

17. Verfahren nach Anspruch 16 zum Herstellen einer Verbindung der Formel II, worin der Arylbestandteil unsubstituiert ist (q ist 0), X ein Wasserstoffatom, eine Methyl- oder Ethylgruppe ist, n 1 oder 2 ist und Yⁿ⁻ Chlorid, Bromid, lodid, ein Sauerstoffsäureanion oder ein Anion einer organischen Säure ist.

18. Verfahren nach Anspruch 16 zum Herstellen einer Verbindung der Ansprüche 16 oder 17, worin der Arylbestandteil in der C-6-Stellung in dem Ring C monosubstituiert ist (wenn q 1 ist) oder unsubstituiert ist (wenn q 0 ist), R ein Wasserstoffatom, eine Methoxy- oder Dimethylaminogruppe ist, R₁ Wasserstoff ist, R₂ ein Wasserstoffatom oder eine Alkylgruppe mit 1 bis 6 Kohlenstoffatomen in linearer, verzweigter oder cyclischer Konfiguration ist.

19. Verfahren nach Anspruch 16 zum Herstellen von 1,4-Bis(n-butylamino)-3(2)H-benzo[g]phthalaziniumchlorid.

20. Verfahren nach Anspruch 16 zum Herstellen von 1,4-Bis(n-butylamino)-6-methoxy-3(2)H-benzo[g]phthalaziniumchlorid.

21. Verfahren nach Anspruch 16 zum Herstellen von 1,4-Bis(n-propylamino)-3(2)H-benzo[g]phthalaziniumchlorid.

22. Verfahren nach Anspruch 16 zum Herstellen von 1,4-Bis(n-propylamino)-3(2)H-6-methoxy-benzo[g]phthalaziniumchlorid.

23. Verwendung einer Verbindung nach einem der Ansprüche 1 bis 15 zum Herstellen eines Arzneimittels zum Behandeln von Krebs.

24. Verwendung einer Verbindung nach einem der Ansprüche 1 bis 15 zum Herstellen eines Arzneimittels zum Behandeln der Chagas-Krankheit.

25. Verwendung einer Verbindung nach einem der Ansprüche 1 bis 15 zum Herstellen eines Arzneimittels zum Behandeln von Trypanosomiasis und anderen Protozoen-Krankheiten.

## Patentansprüche (Patentansprüche für folgende(n) Vertragsstaat(en): ES)

1. Verfahren zum Herstellen einer Verbindung mit der Formel 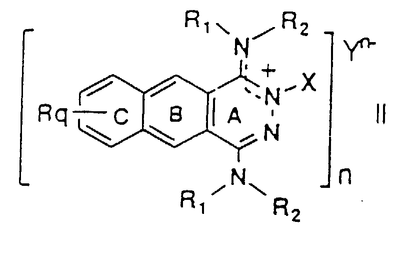 worin
der Arylbestandteil in (einer) beliebigen geeigneten Stellung bzw. Stellungen in dem Ring C mono- oder disubstituiert ist (wenn q 1 bzw. 2 ist) oder unsubstituiert ist (wenn q 0 ist),
R ein niederes Alkyl, bei dem es sich um eine verzweigte oder lineare Alkylgruppe mit 1 bis 6 Kohlenstoffatomen handelt, Aryl, Alkoxy, Nitro, Amino, Alkylamino, Dimethylamino oder Acylamino ist,
R₁ und R₂ gleich oder voneinander verschieden sind und jeweils Wasserstoff, ein niederes Alkyl, bei dem es sich um eine verzweigte oder lineare Alkylgruppe mit 1 bis 6 Kohlenstoffatomen handelt, Aryl, Aralkyl, Cycloalkyl, Cycloalkylalkyl sind, oder R₁ und R₂ zusammen mit dem Stickstoff, an den sie gebunden sind, Pyrrolidino oder Piperidino sind, n 1 oder 2 ist, X Wasserstoff, eine Methyl- oder Ethylgruppe ist, und Yⁿ⁻ Chlorid, Bromid, lodid, ein Sauerstoffsäureanion oder ein Anion einer organischen Säure ist,
welches das Umsetzen eines 1,4-Bis(amino)benzo[g]phthalazins mit der Formel 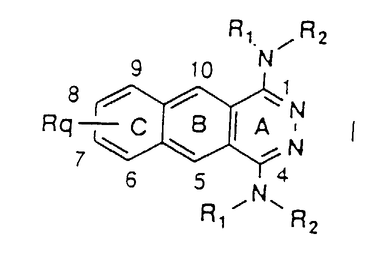 worin R_{q}, R₁ und R₂ wie oben definiert sind, mit Säuren wie Chlorwasserstoffsäure, Schwefelsäure und dergleichen oder Alkylhalogeniden wie Methyl- oder Ethylchlorid umfaßt.

2. Verfahren nach Anspruch 1 zum Herstellen einer Verbindung der Formel II, worin der Arylbestandteil unsubstituiert ist (q ist 0), X ein Wasserstoffatom, eine Methyl- oder Ethylgruppe ist, n 1 oder 2 ist und Yⁿ⁻ Chlorid, Bromid, lodid, ein Sauerstoffsäureanion oder ein Anion einer organischen Säure ist.

3. Verfahren nach Anspruch 1 zum Herstellen einer Verbindung der Ansprüche 1 oder 2, worin der Arylbestandteil in der C-6-Stellung in dem Ring C monosubstituiert ist (wenn q 1 ist) oder unsubstituiert ist (wenn q 0 ist), R ein Wasserstoffatom, eine Methoxy- oder Dimethylaminogruppe ist, R₁ Wasserstoff ist, R₂ ein Wasserstoffatom oder eine Alkylgruppe mit 1 bis 6 Kohlenstoffatomen in linearer, verzweigter oder cyclischer Konfiguration ist.

4. Verfahren nach Anspruch 1 zum Herstellen von 1,4-Bis(n-butylamino)-3(2)H-benzo[g]phthalaziniumchlorid.

5. Verfahren nach Anspruch 1 zum Herstellen von 1,4-Bis(n-butylamino)-6-methoxy-3(2)H-benzo[g]phthalaziniumchlorid.

6. Verfahren nach Anspruch 1 zum Herstellen von 1,4-Bis(n-propylamino)-3(2)H-benzo[g]phthalaziniumchlorid.

7. Verfahren nach Anspruch 1 zum Herstellen von 1,4-Bis(n-propylamino)-3(2)H-6-methoxy-benzo[g]phthalaziniumchlorid.

## Revendications (Revendications pour l'(les) Etat(s) contractant(s) suivant(s): AT, BE, CH, DE, DK, FR, GB, IT, LI, LU, NL, SE)

1. Composé de la formule : 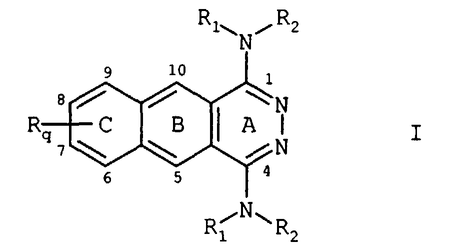 dans laquelle
le fragment aryle est mono- ou disubstitué dans n'importe quelle(s) position(s) possible(s) du cycle C (lorsque q est égal à 1 ou 2, respectivement) ou bien non substitué (lorsque q est égal à 0),
R est un groupe alkyle inférieur qui est un groupe alkyle ramifié ou linéaire de 1 à 6 atomes de carbone, aryle, alcoxy, nitro, amino, alkylamino, diméthylamino ou acylamino,
R₁ et R₂ sont identiques ou différents et sont chacun un groupe alkyle inférieur qui est un groupe alkyle ramifié ou linéaire de 1 à 6 atomes de carbone, aryle, aralkyle, cycloalkyle, cycloalkylalkyle, ou bien R₁ et R₂ pris ensemble avec l'azote auquel ils sont liés sont un groupe pyrrolidino ou pipéridino ou un sel de 1,4-bis (amino)-benzo[g]phtalazinium de la formule : 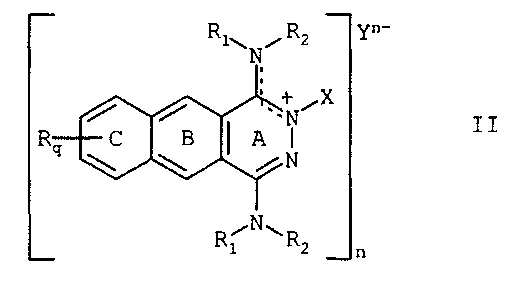 dans laquelle R_{q}, R₁ et R₂ ont les significations indiquées ci-dessus, n vaut 1 ou 2, X est un groupe hydrogène, méthyle ou éthyle, et Yⁿ⁻ est un chlorure, un bromure, un iodure, un anion d'oxacide ou un anion d'acide organique.

2. Composé selon la revendication 1, de formule I dans laquelle le fragment aryle est non substitué (q = 0), R₁ est un hydrogène et R₂ est un groupe hydrogène, alkyle inférieur qui est un groupe alkyle ramifié ou linéaire de 1 à 6 atomes de carbone, aryle, aralkyle, cycloalkyle ou cycloalkylalkyle.

3. Composé selon la revendication 1, de formule I dans laquelle le fragment aryle est monosubstitué (q = 1) au niveau du carbone en C-6, et R est un groupe alkyle inférieur qui est un groupe alkyle ramifié ou linéaire de 1 à 6 atomes de carbone, aryle, alcoxy, nitro, amino, alkylamino, diméthylamino ou acylamino.

4. Composé selon la revendication 1 ou la revendication 3, de formule I dans laquelle R₁ est un hydrogène et R₂ est un groupe hydrogène, alkyle inférieur qui est un groupe alkyle ramifié ou linéaire de 1 à 6 atomes de carbone, aryle, aralkyle, cycloalkyle ou cycloalkylalkyle.

5. Composé selon la revendication 1, de formule II dans laquelle le fragment aryle est non substitué (q = 0), X est un groupe hydrogène, méthyle ou éthyle, n vaut 1 ou 2, et Yⁿ⁻ est un chlorure, un bromure, un iodure, un anion d'oxacide ou un anion d'acide organique,

6. Composé selon l'une quelconque des revendications 1 à 5, dans lequel le fragment aryle est monosubstitué dans la position C-6 du cycle C (lorsque q = 1) ou non substitué (lorsque q = O), R est un groupe méthoxy ou diméthylamino, R₁ et R₂ sont chacun un hydrogène, un groupe alkyle ayant de 1 à 6 atomes de carbone dans une configuration linéaire, ramifiée ou cyclique.

7. 1,4-bis (n-butylamino)benzo[g]phtalazine.

8. Chlorure de 1,4-bis(n-butylamino)-3(2)H-benzo[g]phtalazinium.

9. 1,4-bis(n-butylamino)-6-méthoxybenzo[g]phtalazine.

10. Chlorure de 1,4-bis(n-butylamino)-6-méthoxybenzo[g] phtalazinium.

11. 1,4-bis(n-propylamino)benzo[g]phtalazine.

12. Chlorure de 1,4-bis (n-propylamino)-3(2)H-benzo[g] phtalazinium.

13. 1,4-bis (n-propylamino)-6-méthoxybenzo[g]phtalazine.

14. Chlorure de 1,4-bis(n-butylamino)-6-méthoxy-3(2)H-benzo[g]phtalazinium.

15. Composition pharmaceutique contenant un composé selon l'une quelconque des revendications précédentes dans un mélange avec un véhicule pharmaceutiquement acceptable.

16. Procédé de préparation d'un composé de formule : 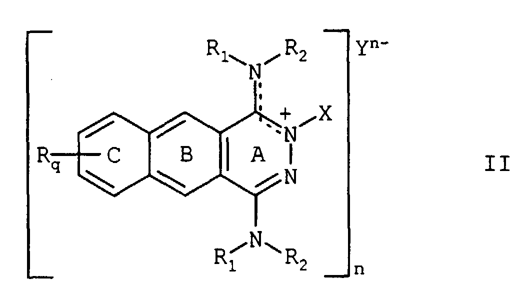 dans laquelle
le fragment aryle est mono- ou disubstitué dans n'importe quelle(s) position(s) possible(s) du cycle C (lorsque q est égal à 1 ou 2, respectivement) ou bien non substitué (lorsque q est égal à 0),
R est un groupe alkyle inférieur qui est un groupe alkyle ramifié ou linéaire de 1 à 6 atomes de carbone, aryle, alcoxy, nitro, amino, alkylamino, diméthylamino ou acylamino, R₁ et R₂ sont identiques ou différents et sont chacun un groupe alkyle inférieur qui est un groupe alkyle ramifié ou linéaire de 1 à 6 atomes de carbone, aryle, aralkyle, cycloalkyle, cycloalkylalkyle, ou bien R₁ et R₂ pris ensemble avec l'azote auquel ils sont liés sont un groupe pyrrolidino ou pipéridino, n vaut 1 ou 2, X est un groupe hydrogène, méthyle ou éthyle, et Yⁿ⁻ est un chlorure, un bromure, un iodure, un anion d'oxacide ou un anion d'acide organique,
ledit procédé consistant à faire réagir une 1,4-bis(amino)-benzo[g]phtalazine de la formule : 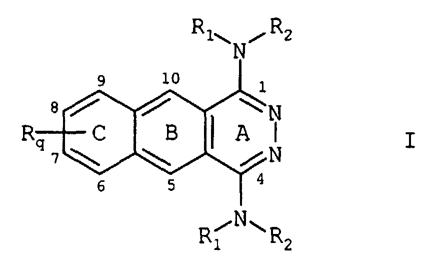 dans laquelle R_{q}, R₁ et R₂ ont les significations indiquées ci-dessus, avec des acides tels que l'acide chlorhydrique, l'acide sulfurique et similaires ou des halogénures d'alkyles tels que le chlorure de méthyle ou d'éthyle.

17. Procédé selon la revendication 16 pour préparer un composé de la formule II dans laquelle le fragment aryle est non substitué (q = 0), X est un atome d'hydrogène, un groupe méthyle ou éthyle, n vaut 1 ou 2, et Yⁿ⁻ est un chlorure, un bromure, un iodure, un anion d'oxacide ou un anion inorganique.

18. Procédé selon la revendication 16 pour préparer un composé selon les revendications 16 ou 17 dans lequel le fragment aryle est monosubstitué dans la position C-6 du cycle C (lorsque q = 1) ou non substitué (lorsque q = O), R est un groupe méthoxy ou diméthylamino, R₁ est un atome d'hydrogène, R₂ est un atome d'hydrogène ou un groupe alkyle ayant de 1 à 6 atomes de carbone dans une configuration linéaire, ramifiée ou cyclique.

19. Procédé selon la revendication 16 pour préparer du chlorure de 1,4-bis (n-butylamino)-3(2)H-benzo[g]phtalazinium.

20. Procédé selon la revendication 16 pour préparer du chlorure de 1,4-bis (n-butylamino)-6-méthoxy-3(2)H-benzo [g]phtalazinium.

21. Procédé selon la revendication 16 pour préparer du chlorure de 1,4-bis (n-propylamino)-3(2)H-benzo[g]phtalazinium.

22. Procédé selon la revendication 16 pour préparer du chlorure de 1,4-bis(n-propylamino)-3(2)H-6-méthoxybenzo[g]phtalazinium.

23. Utilisation d'un composé selon l'une quelconque des revendications 1 à 15 pour préparer un médicament destiné au traitement du cancer.

24. Utilisation d'un composé selon l'une quelconque des revendications 1 à 15 pour préparer un médicament destiné au traitement de la maladie de Chagas.

25. Utilisation d'un composé selon l'une quelconque des revendications 1 à 15 pour préparer un médicament destiné au traitement de la trypanosomiase et d'autres affections provoquées par des protozoaires.

## Revendications (Revendications pour l'(les) Etat(s) contractant(s) suivant(s): ES)

1. Procédé de préparation d'un composé de formule : 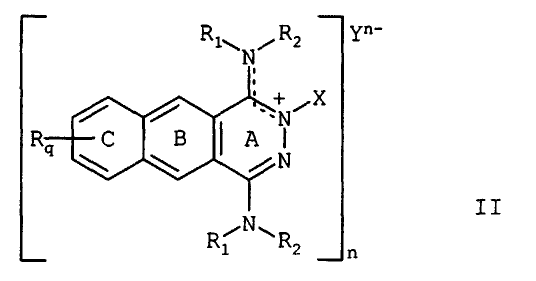 dans laquelle
le fragment aryle est mono- ou disubstitué dans n'importe quelle(s) position(s) possible(s) du cycle C (lorsque q est égal à 1 ou 2, respectivement) ou bien non substitué (lorsque q est égal à 0),
R est un groupe alkyle inférieur qui est un groupe alkyle ramifié ou linéaire de 1 à 6 atomes de carbone, aryle, alcoxy, nitro, amino, alkylamino, diméthylamino ou acylamino, R₁ et R₂ sont identiques ou différents et sont chacun un groupe alkyle inférieur qui est un groupe alkyle ramifié ou linéaire de 1 à 6 atomes de carbone, aryle, aralkyle, cycloalkyle, cycloalkylalkyle, ou bien R₁ et R₂ pris ensemble avec l'azote auquel ils sont liés sont un groupe pyrrolidino ou pipéridino, n vaut 1 ou 2, X est un groupe hydrogène, méthyle ou éthyle, et Yⁿ⁻ est un chlorure, un bromure, un iodure, un anion d'oxacide ou un anion d'acide organique.
ledit procédé consistant à faire réagir une 1,4-bis(amino)-benzo[g]phtalazine de la formule : 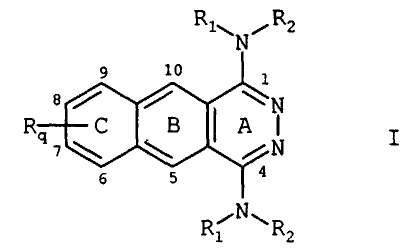 dans laquelle R_{q}, R₁ et R₂ ont les significations indiquées ci-dessus, avec des acides tels que l'acide chlorhydrique, l'acide sulfurique et similaires ou des halogénures d'alkyles tels que le chlorure de méthyle ou d'éthyle.

2. Procédé selon la revendication 1 pour préparer un composé de la formule II dans laquelle le fragment aryle est non substitué (q = 0), X est un atome d'hydrogène, un groupe méthyle ou éthyle, n vaut 1 ou 2, et Yⁿ⁻ est un chlorure, un bromure, un iodure, un anion d'oxacide ou un anion d'acide organique.

3. Procédé selon la revendication 1 pour préparer un composé selon les revendications 1 ou 2 dans lequel le fragment aryle est monosubstitué dans la position C-6 du cycle C (lorsque q = 1) ou non substitué (lorsque q = 0), R est un groupe méthoxy ou diméthylamino, R₁ est un atome d'hydrogène, R₂ est un atome d'hydrogène ou un groupe alkyle ayant de 1 à 6 atomes de carbone dans une configuration linéaire, ramifiée ou cyclique.

4. Procédé selon la revendication 1 pour préparer du chlorure de 1,4-bis(n-butylamino)-3(2)H-benzo[g]phtalazinium.

5. Procédé selon la revendication 1 pour préparer du chlorure de 1,4-bis (n-butylamino)-6-méthoxy-3(2)H-benzo [g]phtalazinium.

6. Procédé selon la revendication 1 pour préparer du chlorure de 1,4-bis(n-propylamino)-3(2)H-benzo[g]phtalazinium.

7. Procédé selon la revendication 1 pour préparer du chlorure de 1,4-bis(n-propylamino)-3(2)H-6-méthoxybenzo[g]phtalazinium.
